# EUROPEAN PATENT APPLICATION

(11) **EP 4 242 622 A1**
(43) Date of publication of application: **13.09.2023**
(21) Application number: 22160938.1
(22) Date of filing: 08.03.2022
(51) Int. Cl.: G01N 1/28, G06Q 50/02, G06Q 30/00, G01N 33/02, G01N 33/543

(54) **TESTING SYSTEM FOR AGRICULTURAL PRODUCE**

(71) Applicant: SwissDeCode SA, 1020 Renens (CH)
(72) Inventor: Varicchio, Stefano, 1003 Lausanne (CH); Breteinmoser, Adrian, 1004 Lausanne (CH); Vergnet, BAstien, 1009 Pully (CH); Bataille, Gregory, 1260 Nyon (CH); Rando, Gianpaolo, 1087 Blonay (CH)
(74) Representative: Vossius & Partner Patentanwälte Rechtsanwälte mbB

(57) **Abstract**

The present invention relates to a system and method for testing agricultural produce such as grain, oilseeds, legume pulses, beans and nuts. The system comprises a testing apparatus for installation at a user's testing site configured to process and test the produce. The testing apparatus comprises one or more sensor devices for recording the testing process and a control unit configured to operate the testing apparatus and transmit the test record and test data conveying a test result. The system further comprises a remote validation system for receiving and validating the test data and test record.

## Description

### Background

The invention relates to a testing system for agricultural produce. In the process of commercializing agricultural produce, said produce must routinely be tested and verified during import and exporting procedures.

One problem in the industry is the analysis of grain samples to verify the genetic content. One specific example is the testing of corn kernels to verify whether they contain genetically modified strains (GMO). For example, the European Union does not accept grain with a GMO content of more than 1%. If the certificate of analysis says the corn contains more than 1% GMO the vessel can be either rerouted to another country (extra costs) or sold in Europe for animal feed production (lower profits).

In the context of grain trading, vessels transport produce in bulk from an origin country to a destination country. In the origin country, at the port, an infrastructure called either a grain elevator or export terminal is responsible to fill the vessel with the right grain requested for trading. During this process, an independent auditor from the testing inspection and certification industry oversees the loading process and takes samples that are sent to an external accredited lab. The produce in transport must be tested for a variety of different parameters such that the quality can be verified. Grain, for example, can be tested for admixture with other grains, GMO, mycotoxin, humidity levels, broken kernels, sprouting kernels, insect damage, etc. Many of these testing steps are performed manually which justifies the need of a laboratory with trained personnel to minimize the risk of errors. A few days later, after receival of the certificates of analysis from the laboratory, the independent testing, inspection and certification company issues a final "vessel certificate" that will be used for trade to confirm the final identity, quality and price of grain. This process can be time consuming, and because the costs associated to vessel demurrage and grain retention are too high, vessels usually leave the port before a proper certification can be completed. This delay in certification exposes the grain industry to risk of litigations, rerouting costs and unnecessary CO2 emissions every time the vessel certificate does not meet the specifications requested by the client in the destination country. To minimize those risks, the grain might be preemptively screened before loading the vessel, in a non-accredited environment adding extra costs and complexities but without solving the need of a faster and more efficient certification system.

It is an object of the present invention to provide a testing system for agricultural produce which provides an improvement over known testing processes and addresses the problems described.

### Summary

This object is achieved with the features of the independent claims. Dependent claims refer to preferred embodiments.

According to a first aspect, the invention relates to a testing system for an agricultural produce in the form of grain, oilseeds, legume pulses, beans and/or nuts. The testing system comprises a testing apparatus for installation at a user's testing site and is configured to process and test the produce.

The testing system, preferably, further comprises a validation system. The validation system preferably is a remote validation system. The testing apparatus preferably comprises one or more sensor devices configured to record the testing process as a test record, and a control unit configured to operate the testing apparatus and transmit the test record and test data relating to a test result. The remote validation system comprises a remote server configured to receive the test data and the test record from the testing apparatus. The remote validation system is configured to validate the test data and the test record and to transmit a validation to the user and/or a third party in the form of a certificate and/or test report. While the test system preferably comprises such remote validation system, it will be understood that the testing apparatus and system disclosed herein may also be useful without such validation system or without the validation system being remote, and may offer still advantages (such as a fully automated and, preferably, validated testing). For example, the validation could be performed on-site, e.g. by the testing apparatus itself, for example if the apparatus is located in an accredited environment.

Preferably the testing system is configured for testing corn. The testing system may also be configured for detecting the presence of genetically modified produce, in particular genetically modified corn. As testing for genetically modified produce is required for some import/export procedures, provision of an automated testing system for GMOs can reduce transport times and costs.

Preferably, the testing apparatus is configured to grind the produce into powder, mix the powder with a liquid to form a mixture, test a sample (e.g., a sub-sample) of the mixture, more preferably a supernatant of the mixture, and transmit the test data and the test record to the remote validation system.

Preferably, the remote validation system is configured to: receive the test data and the test record, analyze the test data, analyze the test record, validate the test data and the test record on the basis of the analyses, and issue and transmit a validation certificate to the user and/or a third party. By validating the test data and the test record it can be ensured that the testing process has taken place appropriately and that the test data are reliable.

Preferably, the testing apparatus is configured to receive an input of at least 100 g of produce, more preferably at least 200 g of produce, even more preferably at least 300 g of produce. The testing apparatus may also be configured to receive an input of at most 10 kg of produce, preferably at most 3 kg of produce, more preferably at most 2.5 kg of produce. The testing apparatus may be configured to utilize at least 100 g, at least 200 g, or at least 300 g of corn for testing. Testing in bulk of produce ensures that the sample is sufficient and representative of the produce being transported.

Preferably, the testing apparatus further comprises a feeder module for providing a randomized sample of the produce filled into the testing apparatus, the randomized sample having a predetermined weight and/or volume. A randomized sample further helps to ensure that the sample is representative of the bulk produce.

Preferably, the testing apparatus further comprises a grinding module for milling the produce, wherein the grinding module comprises a mill for grinding the produce, preferably into a powder.

Preferably, the grinding module further comprises an accumulator arranged before the mill, more preferably above the mill. Even more preferably the produce moves from the accumulator into the mill by gravity. Advantageously, the testing of produce can be more easily performed when the produce is rendered into a small particle format, such as a powder.

Preferably, the testing apparatus further comprises a mixing module for combining produce milled into powder with a liquid to prepare a sample suspension therefrom. A suspension of the produce in, for example, water, enables faster and easier testing.

Preferably, the mixing module comprises a liquid source, at least one container, and a mixing motor. The container receives the powder and the liquid. Preferably, at least one mixing implement is provided for mixing the powder and liquid. The mixing implement may be provided as part of the container.

Preferably, the implement is arranged to be driven by the mixing motor to mix the powder and liquid to create the sample suspension. Advantageously, mixing of the produce powder and liquid with an implement ensures the even distribution of particles and a complete sampling of the produce.

Preferably, the mixing module further comprises a turn table upon which the container is mounted, preferably wherein n containers, n being an integer of at least 2, 3 or 4, are mounted on the turn table. The n containers may be mounted on the turn table in a radially symmetric arrangement, preferably wherein the turn table is rotated by 360°/n degrees when actuated.

Preferably, n is an integer of at least 3, and the containers rotate through at least three stations, including a mixing station, wherein the powder and liquid are received and the suspension is mixed, a testing station wherein the supernatant is collected and tested, and a cleaning station, wherein the container is rinsed. As a particularly advantageous embodiment a turn table with multiple containers enables multiple produce samples to be tested simultaneously with a different produce sample present in each of the containers.

Preferably, the testing apparatus further comprises a testing arrangement for performing a test on the sample suspension. More preferably the testing arrangement further comprises a robotic arm for manipulating one or more test assays. Even more preferably the robotic arm is configured to pick up an assay and introduce the assay into a supernatant of the suspension. Automated testing of the produce sample suspension provides a reliable and repeatable means for the obtaining test data.

Preferably, each assay comprises at least one lateral flow test, more preferably a plurality of lateral flow tests, and even more preferably the plurality of lateral flow tests are provided in a comb arrangement. Lateral flow tests are quick and reliable assays for identification of genetic and protein materials of plants. When provided in a comb arrangement multiple different assays may be performed simultaneously, such as testing for different strains of GMO, mycotoxins and/or allergens.

In some embodiments, one or more test assays comprising at least one nucleic acid amplification test may be employed. For example, the nucleic acid amplification test may be provided using LAMP or PCR testing technology.

Preferably, the control unit further comprises a data processing, encryption and private server/ cloud connection unit for transmitting the test data and the test record. Advantageously, a secure connection between the control unit and the remote validation system precludes that other parties may interfere with the data being either transmitted or received. Thus, the reliability of the validation process is ensured.

According to a further aspect, the invention is also directed to a method for testing and validating an agricultural produce in the form of grain, oilseeds, legume pulses, beans and/or nuts at a user's testing site, the method comprising the steps of:
i. receiving an amount of produce within a testing apparatus;
ii. processing the produce with the testing apparatus;
iii. testing the produce with the testing apparatus;
iv. recording a testing record and a test data relating to the test result;
v. transmitting the test data and the test record to a remote validation system;
vi. validating the test data and the test record using the remote validation system; and
vii. transmitting the validation to the user and/or a third party.

Preferably, processing the grain includes measuring a predetermined amount of grain from the input amount, grinding the predetermined amount of grain into powder, and mixing the powder with a liquid to create a suspension. Preferably, processing the grain further includes sampling a supernatant of the suspension.

Preferably, testing the produce includes introducing an assay into the suspension, more preferably into a supernatant of the suspension.

Preferably, transmitting the test data further comprises encrypting and sending the test record and test data using a private cloud connection.

### Brief Description of the Drawings

The subject matter of the invention will be explained in more detail in the following text with reference to preferred exemplary and non-limiting embodiments which are illustrated in the attached drawings. These figures disclose embodiments of the invention for illustrational purposes only. In particular, the disclosure provided by the figures and description is not meant to limit the scope of protection conferred by the invention.
Fig. 1 illustrates the features and process flow of the testing system;
Fig. 2a schematically shows a cross-sectional view of a feeder module presented at an angle;
Fig. 2b schematically shows a side view of the feeder module as shown in Fig. 2a.
Fig. 3a schematically shows an angled view of a controlled sampling device in a configuration where produce can be output for testing;
Fig. 3b schematically shows the controlled sampling device of Fig. 3a after enough produce has been provided for testing and the excess produce is redirected to an alternative outlet;
Fig. 4a schematically depicts a grinding module;
Fig. 4b schematically shows a cross-section of an accumulator and a mill of the grinding module;
Fig. 5a schematically depicts an embodiment of a mixing module at an angle;
Fig. 5b schematically depicts the mixing module of Fig. 5a in a side view cross-section;
Fig. 6a schematically depicts a container and supernatant collector in an angled view;
Fig. 6b schematically depicts the container and supernatant collector in a perspective cut-away view;
Fig. 7 schematically illustrates an embodiment of the mixing module comprising a turn table;
Figs. 8a-8c schematically show an implementation of the testing arrangement; and
Fig. 9 schematically depicts an embodiment of a cleaning module.

### Detailed Description

In the following embodiments, specific features and a use of a system in accordance with the invention will be described in connection with corn kernels for ease of explanation. However, this should not be understood as a limitation of the system. Instead, it will be understood by a person skilled in the art that the inventive testing system 10 herein described can be used in combination with a wide variety of types of agricultural produce. Specific examples of suitable agricultural produce include grain, oilseeds, legume pulses, beans and/or nuts, for example. The device may also be scaled up and down in size to accept these different types of inputs.

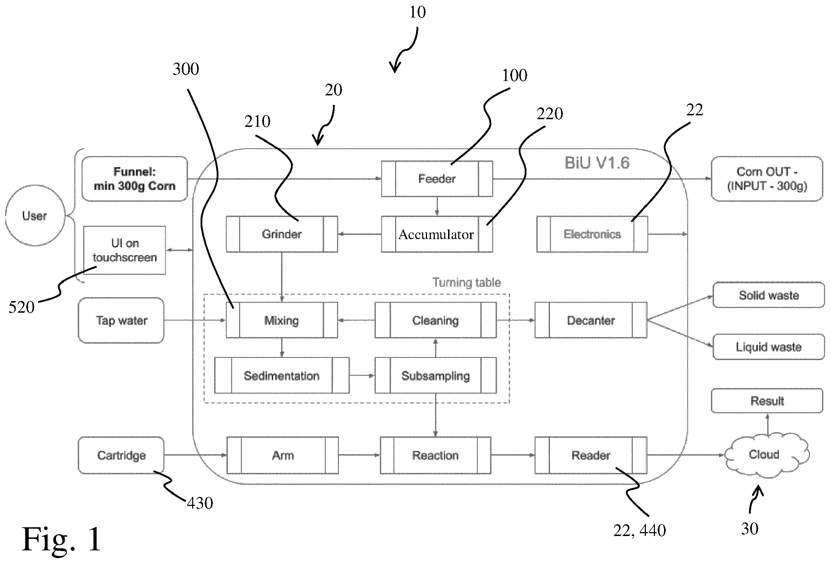

Fig. 1 shows a flow-chart of the testing apparatus 20 of the testing system 10, wherein the physical testing apparatus 20 is encircled and inputs and outputs of the testing apparatus 20 are shown outside the circle. The testing apparatus 20 is located at a user's testing site, which depending on circumstances may be a testing site, a produce loading site, a produce storage site, or any combination thereof. When a user in possession of the present testing apparatus 20 wishes to test a portion of corn the user can input an amount of corn into the testing apparatus 20.

While the amount of produce input into the apparatus will vary based on the type of produce being input, the guiding principal is the inclusion of enough discrete samples such that a reliable batch measurement can be made. Thus, the testing apparatus 20 may be configured to receive an input of at least 100 g, 200 g, 300 g, or more such that a large enough sample is obtained. Maximally the apparatus may be configured to receive 10 kg, 3 kg, 2.5 kg or less. In the specific example of corn, each kernel weighs around 0.2 to 0.3 g, if an upper end of that range is assumed, a sample of around 300 g would provide around 1000 individual corn kernels, i.e. individual corn samples. This same guiding principal can be applied to other kinds of produce given their weight and the statistical accuracy required of the result.

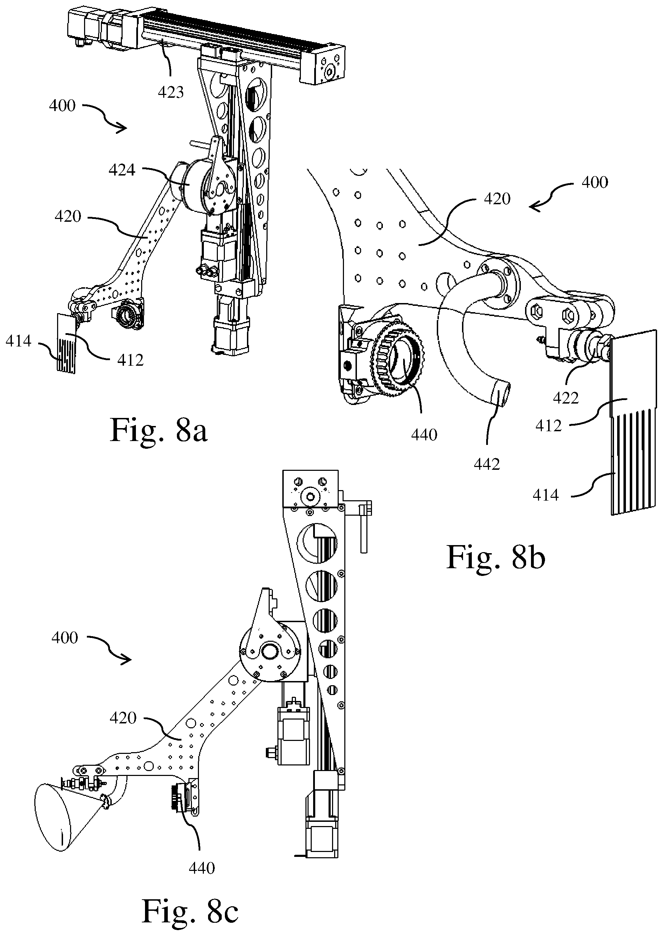

In order to eliminate the need for a user to weigh out the amount of produce being input, the user may input any amount over the minimum required amount and the input can be weighed by the system and the excess discarded. As shown in Fig. 1, the user inputs a minimum of 300 g of corn into the testing apparatus 20 which is then delivered to a feeder module 100. The feeder module 100 can be used to input the corn in a regulated manner in terms of weight and flow rate. From the feeder module 100 the corn can be fed into an accumulator 220, which serves to direct the corn kernels into the grinder/mill 210. The mill 210 pulverizes the corn kernels into a powder which is then transported to the mixing module 300, wherein the powder is mixed with a liquid, preferably water. This mixture can then be sampled with an assay 412 (see Figs. 8a to 8c) where the result is transmitted to the remote validation system 30, which comprises a remote server.

In the following, each component of the testing system 10 will be described in more detail. The testing apparatus 20 as herein described is envisioned as an enclosed device, which can be transported to a user or a testing station 362. In some configurations the testing apparatus 20 may also be modular, in that it can be separated into two or more parts and transported separately. Individual components of the testing apparatus 20 may be purchased or fabricated with techniques such as metal casting, plastic molding, and 3D-printing.

All components of the testing apparatus described below may be enclosed by a connected and/or a common housing (not shown). In particular, at least the feeder module 100, the mixing module 300, and the testing arrangement 400 are preferably enclosed by a common housing and/or connected housings. Such housing(s) may be useful to avoid unallowed access to the components arranged therein and/or tampering with the test.

Some embodiments of the testing apparatus 20 may include a user interface 520 through which operation of the testing apparatus 20 can be controlled. The user interface 520 may be located on a display on the testing apparatus 20 itself or may be displayed on a separate screen. The user interface 520 may be configured to provide instructions to a user and/or to deliver error messages. In some embodiments the user interface 520 may also display a validation once this has been obtained.

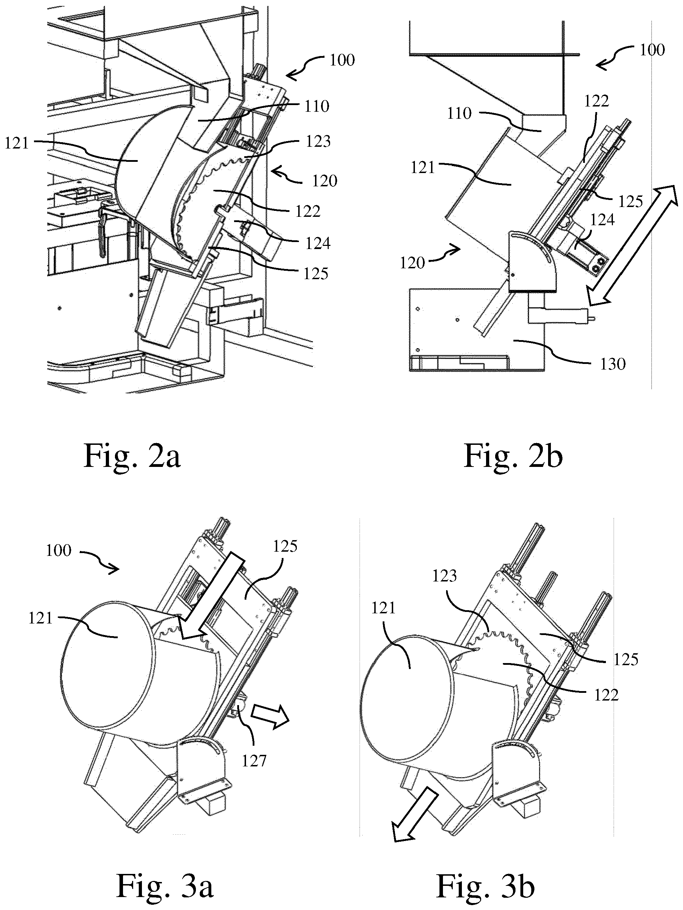

Figs. 2a and 2b depict one embodiment of the feeder module 100. Generally, the feeder module 100 is configured to provide an amount of corn to the grinding module 200 in a controlled manner. The feeder module 100 includes a controlled sampling device 120 and preferably an excess collection device 130.

The feeder module 100 may further include a funnel 110. The funnel 110 may be positioned to receive corn from outside of the testing apparatus 20 and direct it to the controlled sampling device 120.

The controlled sampling device 120 is configured to provide the corn kernels to the grinding module 200 at a steady rate such that the mill 210 is not overwhelmed or clogged. In the embodiment depicted in Fig. 2a, the controlled sampling device 120 comprises a backboard 125, and a wheel 122 which is mounted adjacent the backboard 125. The wheel 122 may preferably be placed parallel to the backboard 125.

The controlled sampling device 120 may also comprise a housing 121 which is mounted on the backboard 125. The housing 121 may have any number of shapes. In the present embodiment the housing 121 is cylindrically shaped having one flat face, one curved face, and an opening in the curved face, wherein the flat face has a diameter slightly larger than the diameter of the wheel 122. The housing 121 and the backboard 125 together form a container 320 wherein the corn kernels are received from the funnel 110 and held. The wheel 122 is positioned between the backboard 125 and the housing 121 and comprises gaps 123 around the circumference of the wheel 122. The gaps 123 may have any appropriate form or interval such that each gap 123 has space for a small amount of corn kernels, legume pulses, nuts, etc, preferably for a predetermined amount such as 5 or less, 3 or less, or 1. The gaps 123 need not be at the perimeter of the wheel 122 and instead may be holes in the face of the wheel 122 for some implementations.

The backboard 125 in this embodiment is positioned at an angle relative to horizontal such that the corn kernels, when present within the housing 121 are held against and/or slide into the wheel 122 by gravity. This ensures that kernels are continually positioned within the gaps 123 of the wheel 122. A wheel motor 124 is configured to drive the wheel 122 such that it rotates about the center. The wheel motor 124 may be positioned on the side of the backboard 125 opposite the housing 121 and extend through the backboard 125 to make contact with the center of the wheel 122. When the testing apparatus 20 is actuated and corn has been provided, the wheel motor 124 turns the wheel 122 and the corn kernels positioned within the gaps of the wheel 122 are transported circumferentially until they reach an output 127 of the controlled sampling device 120. This output 127 is preferentially formed in the backboard 125 such that kernels fall through due to gravity. Alternatively or additionally, suction may be applied. In this manner a continual flow of single corn kernels is provided to the output.

In some configurations the feeder module 100 also comprises a scale (not shown) and/or an excess collection device 130. The scale may be positioned in a number of places in order to weigh the amount of corn input into the feeder module 100 and/or the amount of corn output from the feeder module 100. For example, the scale may weigh the amount of corn input into the feeder module 100 as well as the amount of corn remaining therein in order to calculate the output.

In some embodiments of the feeder module 100, the entire controlled sampling device 120 is mounted on a scale such that the input amount of corn and the output amount of corn can be monitored. When the target output amount of corn is reached, the controlled sampling device 120 may discharge the excess corn to the excess collection device 130. In this embodiment, when the user inserts the totality of the corn, the scale initially measures the total weight and verifies that its quantity is at least 300 g. If this is not the case, the testing apparatus 20 simply will not start and/or will provide an error message to the user. If the amount of corn is enough, the feeder proceeds to subsample 300 g and feeds said subsample to the next stage. One example of such a mechanism is shown in Fig. 3, wherein initially, as shown in Fig. 3a, the corn is input into the controlled sampling device 120 and the wheel 122 delivers the corn to the output. After the scale determines that the predetermined amount of corn has been reached the control unit (not shown) actuates the housing 121 to descend along the backboard 125 such that the excess corn may escape through an alternative outlet. This operation may be controlled through a linear actuator mounted on the backboard 125, e.g. in combination with two magnetic sensors. The excess corn may then be collected in the excess collection device 130. Alternatively, when the predetermined amount of corn has been delivered to the output of the controlled sampling device 120, the control unit may instruct the wheel motor 124 to drive the wheel 122 in the reverse direction such that the corn reaches an alternative outlet.

The predetermined amount of produce will vary based on the type of produce being used. When corn is used the predetermined amount may be between 100 g and 1000 g, preferably between 200 g and 800 g, more preferably around 300 g.

In order to optimize the process for both accuracy and speed, a first amount of corn (e.g., the first 250 g) may be subsampled with the rotating wheel 122 moving faster while for a second amount of corn (e.g., the last 50 g) the wheel 122 may slow down to provide a more accurate result. This may minimize the operation time.

The rotation of the wheel 122 randomizes the subsample as well, making sure it is representative of the corn added by the user. Once 300g of corn has been subsampled, the feeder returns the corn in excess to the user, e.g. by opening its bottom with a sliding movement. To remove any kernels that might be inside the gaps of the wheel 122, the wheel 122 may be run in the opposite direction when the feeding module is in the open configuration. The excess corn is thereby returned to the user.

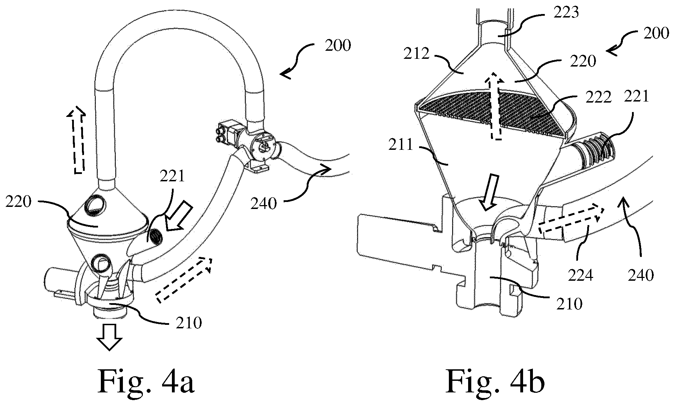

Figs. 4a and 4b illustrate one embodiment of the grinding module 200 which is configured to process the corn from discrete kernels into a powder. In the present embodiment, the output of the feeder module 100 is connected to a conduit (e.g., tubing) in communication with a negative pressure source, i.e. a vacuum. The grinding module 200 may, in some configurations, include an accumulator 220. The accumulator 220 may be used to separate the corn kernels from the airflow and thereby prevent them from reaching the negative pressure source. In Fig. 4a and Fig. 4b, flow of produce is shown with arrows, while the flow of air due to negative pressure is shown with dashed arrows.

As shown in Fig. 4a, a lower part of the accumulator 220 may be shaped as a funnel 110, wherein the narrow part of the funnel 211 receives corn through an inlet 221 and directs corn to a mill 210. An upper part of the accumulator 220 may be shaped as a reverse funnel 212 positioned on top of the lower section, wherein the narrow portion of the reverse funnel 212 forms a first vacuum outlet 223 which forms a connection of the accumulator 220 to the negative pressure source 240.

Positioned below the accumulator 220 is the mill 210, which grinds the produce into a power for further use. A lower opening of the grinder allows the powder to fall through to the mixing module 300.

Details of the accumulator 220 and the grinder may be observed in Fig. 4b, which shows a cross section of the grinding module 200. In between the lower section and the upper section a mesh 222 may be provided to help further prevent material from reaching the negative pressure source. The mesh 222 may have a pore size dependent on the type of produce being processed, for corn a preferred pore size is 3mm x 3 mm.

In this embodiment the mill 210 is positioned below the grinder such that kernels may fall directly into the mill 210. As the feeder module 100 provides corn to the accumulator 220 and the grinder at a steady rate, the kernels fall through the bottom of the accumulator 220 into the mill 210. The mill 210 may be any kind of mill 210 suitable for producing a powder out of the produce provided. One preferred type of mill 210 may be similar to a rotative coffee grinder.

In some embodiments the mill 210 may be configured to produce particles generally smaller than 1000 µm. In some embodiments the mill 210 produces less than 50% of particles having a particle size greater than 1000 µm, preferably less than 20% of particles having a particle size greater than 1000 µm.

Once the corn has been ground, it may occur that a few kernels persist within the accumulator 220 which can lead to cross-contamination between produce samples. Thus, once the grinding has stopped the negative pressure source 240 may be directed toward a second vacuum outlet 224 of the accumulator 220. The second vacuum outlet 224 may be positioned in the accumulator 220 adjacent to the mill 210. Thus, any kernels remaining at this stage may be removed by the negative pressure airflow.

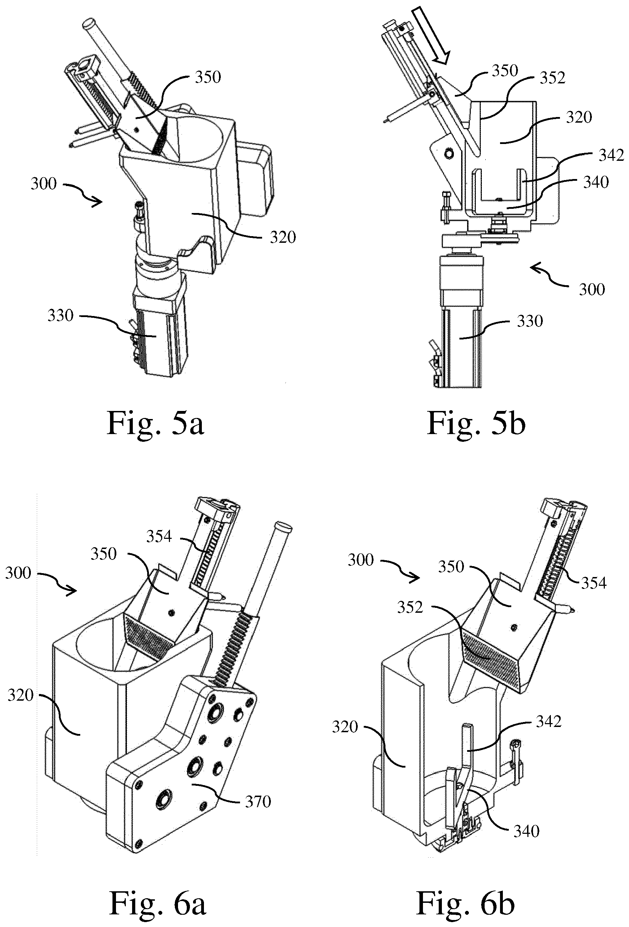

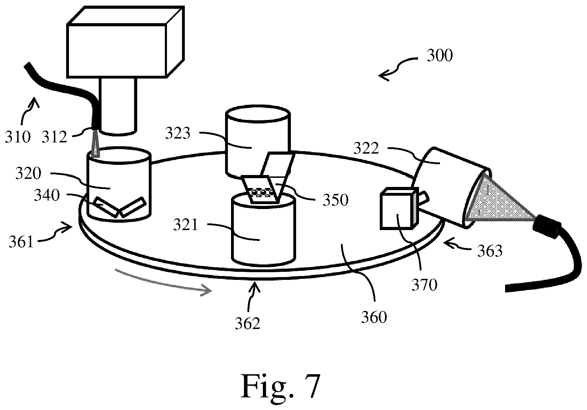

Figs. 5a, 5b, 6a, and 6b illustrate an example embodiment of the mixing module 300. The mixing module 300 may be positioned under the grinding module 200 such that powder produced there may fall from the mill 210 into a container 320. Fig. 5a depicts an example of said container 320, where corn powder may fall into the open end of the container 320 from above. A liquid, such as water, from a liquid source 310 may be delivered through a nozzle 312 (see Fig. 7) into the open end of the container 320.

Within the container 320, as seen in Fig. 5b and 6b a mixing implement 340 may be provided to mix the powder and the liquid. The mixing implement 340 may have at least one, preferably two or more blades 342 which rotate within the container 320. The blades 342 may be configured to scrape the sides and/or a bottom of the container 320 when rotating, e.g. to ensure that the powder and the liquid are adequately mixed. The mixing implement 340 may be driven by a mixing motor 330. Mixing in some embodiments may be partially aided by the placement of the nozzle 312 and the pressure with which liquid is sprayed by the nozzle 312. In some configurations, multiple nozzles 312 can be implemented for injecting the liquid into the container 320, preferably wherein the one or more nozzles 312 are arranged around a periphery of the container.

The amount of liquid provided relative to the amount of powder depends upon the type of produce being tested. For corn, 300g of corn powder may be combined with 450 mL of water.

The liquid may be delivered to the one or more nozzles at a pressure of at least 1 bar, preferably at least 2 bar, more preferably in the range of 3 to 4 bar.

In order to obtain a sample for testing from the mixture, the container 320 may comprise a supernatant collector 350 including a mesh filter 352. The supernatant collector 350 may be actuated to descend into the container 320. The supernatant collector 350 may be configured to obtain a sample of at least 1 mL of supernatant, preferably at least 2 mL of supernatant, more preferably around 2.5 mL of supernatant. The mesh filter 352 allows the supernatant collector 350 to exclude larger particles from the mixture which can interfere with test assay 412s. Figs. 6a and 6b illustrate one embodiment of the supernatant collector 350 in which the supernatant collector 350 is scoop-shaped and affixed to the container 320 at an angle. A collector actuator 354 is affixed to the supernatant collector 350 and configured to linearly descend the supernatant collector 350 into the container 320.

The mesh filter 352 may be made of any suitable material. In one embodiment the mesh filter 352 is formed from a metallic sheet, e.g. having holes 0.8 mm in diameter.

Fig. 7 illustrates an embodiment of the mixing module 300 which further comprises a turn table 360. In such an arrangement, multiple containers 320 to 323 may be present. The turn table 360 may comprise multiple stations for different processing tasks, such as a mixing station 361 wherein the powder and the liquid are combined, a testing station 362 wherein testing with an assay 412 is performed, and a cleaning station 363 wherein the container 320 can be rinsed out for further use. At the mixing station 361 the nozzle 312 delivers water into the container 320, the mixing implement 340 may then be activated for a predetermined amount of time or until sensors determine that the powder and fluid are sufficiently mixed. At the testing station 362 the supernatant collector 350 can then descend into the open end of the container 320 and obtain a sample of the mixture. Further at the testing station 362 a robotic arm 420 (or another actuator) may be implemented in order to administer the assay 412. Finally, at the cleaning station 363 the cleaning actuator 370 may actuate, tipping the container 320 over and allowing it to be rinsed out with water. It will be understood than any effective cleaning method may be employed.

An drying station 323 may be implemented or omitted.

In should be noted that in the embodiment of the mixing module 300 with the turn table 360, each container 320 may comprise a supernatant collector 350 which is fixed thereupon and activated at the testing station 362. Alternatively, a single supernatant collector 350 may be present at the testing station 362.

Similarly, a cleaning actuator 370 may be affixed to each container 320 or a single cleaning actuator 370 may be present at the cleaning station 363.

Similarly, a single mixing motor 330 may be present at the mixing station 361 or a mixing motor 330 may be provided with each container 320.

While four containers 320-323 and three stations are depicted in Fig. 7, it should be noted that any number of containers 320 to 323 may be mounted upon the turn table 360. Similarly, should need arise, additional stations may be provided for the containers 320 to 323 and the rotation of the turn table 360 may be adjusted accordingly. The turn table 360 in combination with multiple containers 320 to 323 and multiple stations allows for multiple tests to be run simultaneously without any cross-contamination.

Figs. 8a-8c depict one embodiment of the testing arrangement 400. Therein a robotic arm 420 is observed which is slidable along a lateral rail 423 and rotatable about a pivot point 424. This combination of these movement allows the robotic arm 420 to collect an assay 412 from an assay cartridge (not pictured) and then introduce the assay 412 into the supernatant sample. It will be understood that other actuators or other robotic arms may be devised.

The actuator or robotic arm 420 may have at the end a manipulator 422, such as, for example, a suction cup or a set of pincers, which are able to securely hold the assay 412. In the case of the robotic arm 420, the robotic arm 420 can then slide along the lateral rail 423 and then pivot about the pivot point 424 such that the assay 412 is introduced into the supernatant within the supernatant collector 350. Depending on the type of assay 412 being performed, a waiting time may be required for the assay 412 to be completed.

Many different types of assay 412 can be used with this system. One particularly useful type of assay 412 is a flow test, e.g. a lateral flow test 414. In some embodiments, each assay 412 may comprise at least one lateral flow test, preferably wherein several lateral flow tests. Several lateral flow tests may be arranged as a plurality of strips (e.g., on a common same substrate). The assay 412, e.g. such an arrangement of strips, may resemble a comb as depicted in Fig. 8a and Fig. 8b. In such an embodiment, the wait time for the assay 412 may be a few minutes.

Flow tests are convenient for the purposes of the invention because they may be stored in the apparatus in the required number and/or for a considerable amount of time. The maintenance requirements of the apparatus may thus be minimized.

After the waiting time, the assay 412 is withdrawn from the container 320 and the assay 412 can then be observed with a read-out arrangement 440. Again, the type of assay 412 being performed will determine the type of read-out arrangement 440 required. In the case of the lateral flow assay 412 described above, the read-out arrangement 440 may be an imaging device. In certain configurations an illuminator 442 may be necessary for the proper read-out of the assay 412. Consequently, the illuminator 442 may shine a light on the assay 412 and the imaging device will then take a digital image of the result which is then provided to the control unit.

In some embodiments the assay 412 may be provided within an assay cartridge 430 (see Fig. 1) for loading onto the robotic arm 420. The assay cartridge 430 may include a biasing mechanism such that when an assay 412 is removed, the next assay 412 is moved into position for use. Such an assay cartridge may allow to store a large number of assays 412, thus minimizing maintenance requirements.

It should be noted that Fig. 1 shows the cartridge 430 outside the apparatus 20 since it may be refilled from the outside during maintenance. It should be noted, however, that the cartridge 430 may be arranged within the housing of the apparatus. For example, the cartridge may be accessible only once the housing is opened (e.g., similar to an ink cartridge within the housing of a printer).

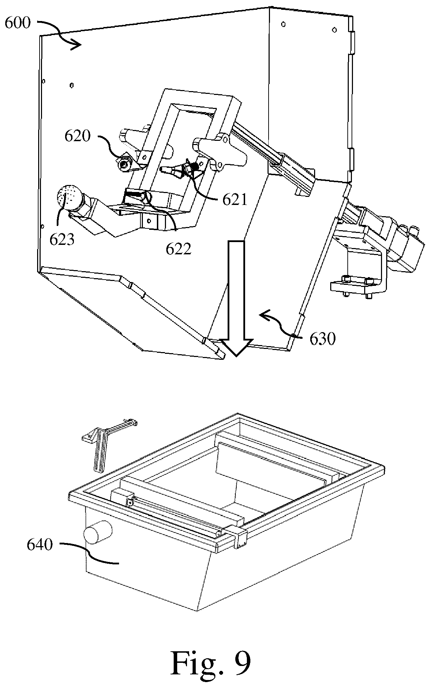

In order for the processing and testing of produce to take place quickly and repeatably, the testing apparatus 20 is advantageously provided with a cleaning module 600 for delivering water and/or cleaning fluid into the container 320 and/or the supernatant collector 350. Fig. 9 illustrates one exemplary embodiment of such cleaning module 600.

When the container 320 is ready to be cleaned after mixing and testing, the cleaning actuator 370 (see Fig. 7) can be engaged such that the container 320 is tipped over and the contents thereof can flow out due to gravity. Alternatively, the bottom of the container could be provided with an outlet.

For the collection of an outflow 630 a decanter 640 may be provided to separate solid waste from liquid waste and to prepare for proper disposal.

The cleaning module 600 may further comprise at least one cleaning nozzle 620 to 623 in connection with a liquid source 610 (e.g., a water source). The at least one cleaning nozzle 620 to 623 may spray liquid into the container 320 and/or onto the supernatant collector 350. For example, the cleaning module 600 may comprise four cleaning nozzles 620 to 623. Three cleaning nozzles 620 to 622 are provided for cleaning the supernatant collector 350, as the mesh filter 352 thereof may be more apt to collect solid particles. The further nozzle 623 may be directed inside the container 320, such that any material remaining on the walls and/or bottom of the container 320 can be removed. The runoff of the spraying process may then also be collected in the decanter 640.

After the container 320 has been sufficiently cleaned, the cleaning actuator 370 may be actuated in the reverse direction, or a biasing mechanism may be employed to return the container 320 into the upright position.

Positioned within the testing apparatus 20 are one or more sensor devices 22 (see Fig. 1). In many cases these sensor devices 22 are imaging devices, but may alternatively be other types of sensors, such as thermal sensors, electrochemical sensors, etc.

At least one of these sensor devices 22 is the read-out arrangement 440 which is configured to record the test data as described above. In some configurations the read-out arrangement 440, being a digital image sensor, is the sensor configured to record the test data. Thus, in some configurations the test data may comprises or is a digital image. In other configurations the test data may further comprise a digital readout or a test result. Additional sensor devices 22 may be positioned within and/or outside of the testing apparatus 20 to record the testing process.

Recording the testing process with, for example, multiple imaging devices ensures that the produce is properly processed, i.e. pulverized, mixed and sampled. Further, said imaging devices also serve to record that the testing process is not tampered with before or during testing.

After the testing process has been recorded in the control unit as a test record, i.e. data provided from one or more sensor devices 22, and the test data has been obtained. This data may then be transmitted by the control unit to the remote validation system 30. This transmission should take place securely. Consequently, the control unit may further include a data processing, encryption and private server / cloud connection unit for secure transmission.

The remote validation system 30 will take place on a remote server which is configured to carry out validation of the test data and the test record. In the case where the read-out arrangement 440 is an imaging device, the validation may include image processing. Image processing may be performed by image analysis and/or AI evaluation of the test data. Some portions of the validation may take place on the control unit before it is transmitted to the remote validation system 30 in order to limit the amount of data which must be transmitted.

Preferably, the test record is also validated by the remote validation system 30, wherein information from the one or more sensor devices 22 other than the read-out arrangement 440 is evaluated with an algorithm to ensure that testing has been carried out appropriately. Validation of the test record and of the test data may be performed with imagine recognition and/or artificial intelligence.

When the validation process is finished, a validation, which may be in the form of certificate and/or test report, may be transmitted back to the user and/or a third party, e.g. automatically or after final validation by a human supervisor.

While the invention has been illustrated and described in detail in the drawings and foregoing description, such illustration and description are to be considered illustrative or exemplary and non-restrictive; the invention is thus not limited to the disclosed embodiments. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality and may mean "at least one".

The following are preferred aspects of the invention:
1. A testing system for an agricultural produce in the form of grain, oilseeds, legume pulses, beans and/or nuts comprising
   a testing apparatus for installation at a user's testing site and being configured to process and test the produce, comprising
      one or more sensor devices configured to record the testing process as a test record,
      a control unit configured to operate the testing apparatus and transmit the test record and test data relating to a test result,
   a remote validation system comprising
      a remote server configured to receive the test data and the test record from the testing apparatus;
   wherein the remote validation system is configured to validate the test data and the test record and to transmit a validation to the user and/or a third party in the form of a certificate and/or test report.
2. The testing system of aspect 1, wherein the testing system is configured for testing corn.
3. The testing system of aspect 1 or 2, wherein the testing system is configured for detecting the presence of genetically modified produce.
4. The testing system of any one of the previous aspects, wherein the testing apparatus is configured to grind the produce into powder, mix the powder with a liquid to form a mixture, test a sub-sample of the mixture, preferably a supernatant of the mixture, and transmit the test data and the test record to the remote validation system.
5. The testing system of any one of the previous aspects, wherein the remote validation system is configured to:
   receive the test data and the test record,
   analyze the test data,
   analyze the test record,
   validate the test data and the test record on the basis of the analyses, and
   issue and transmit a validation certificate to the user and/or third party.
6. The testing system of any one of the previous aspects, wherein the testing apparatus is an enclosed apparatus comprising a feeder module, a grinding module, a mixing module, and a testing arrangement.
7. The testing system of any one of the previous aspects, wherein the testing apparatus is configured to receive an input of at least 100 g of produce, preferably at least 200 g of produce, more preferably at least 300 g of produce, and/or wherein the testing apparatus is configured to receive an input of at most 10 kg of produce, preferably at most 3 kg of produce, more preferably at most 2.5 kg of produce.
8. The testing system of any one of the previous aspects, wherein the testing apparatus is configured to utilize at least 100 g, at least 200 g, or at least 300 g of corn for testing.
9. The testing system of any one of the previous aspects, wherein the testing apparatus further comprises a grain input, a grain output, a liquid input, and a waste output.
10. The testing system of any one of the previous aspects, wherein the testing apparatus further comprises a feeder module for providing a randomized sample of the produce filled into the testing apparatus, the randomized sample having a predetermined weight and/or volume.
11. The testing system of aspect 10, wherein the feeder module includes a funnel adapted to receive an amount of input produce, a controlled sampling device which receives the input produce from the funnel and outputs a predetermined amount of produce, preferably the predetermined amount of produce having a predetermined weight and/or volume, and an excess collection device for receiving all produce in excess of the predetermined amount;
   preferably wherein the controlled sampling device comprises a housing, a wheel comprising intermittent gaps, a wheel motor, and a backboard,
   wherein the wheel is positioned adjacent to the backboard, and the housing and the backboard create an enclosure for the wheel and a holding space for grain,
   wherein the wheel is configured to rotate when driven by the wheel motor, thereby transporting individual grains to an output in the controlled sampling device,
   preferably wherein the backboard is positioned at an angle such that gravity transports the individual grains into the gaps of the wheel.
12. The testing system of aspect 11, wherein the predetermined amount is between 100 and 10000 individual kernels, legumes and/or nuts; preferably between 500 and 2000; more preferably around 1000.
13. The testing system of aspect 11, wherein the predetermined amount is between 100 g and 1000 g, preferably between 200 g and 800 g, more preferably around 300 g.
14. The testing system of any of aspects 11-13, wherein the feeder module further comprises a scale in communication with the control unit for weighing the amount of produce delivered into and/or out of the feeder module, wherein when the predetermined amount of produce has been output the excess produce is redirected to the excess collection device.
15. The testing system of aspect 14, wherein when the predetermined amount is delivered, the housing and/or the backboard are moved with respect to each other until the excess grain falls out through an alternative outlet, preferably wherein the housing descends along the backboard.
16. The testing system of any of aspects 11-15, wherein an airflow, preferably negative pressure, encourages the outflow of the individual grains, legume pulses and/or nuts from the feeder module, and/or wherein an airflow, preferably negative pressure is employed to aid in transporting the produce from the feeder module to a grinding module.
17. The testing system of aspect 16, wherein the testing apparatus comprises at least one vacuum source for generating the negative pressure.
18. The testing system of aspect 16, wherein the testing apparatus further comprises an accumulator for separating the produce from the airflow.
19. The testing system of any one of the previous aspects, wherein the testing apparatus further comprises a grinding module for milling the produce.
20. The testing system of aspect 19, wherein the grinding module comprises a mill for grinding the produce from the feeder module into a powder, preferably wherein the accumulator is arranged between the feeder module and the mill, more preferably above the mill, even more preferably wherein the produce moves from the accumulator into the mill by gravity.
21. The testing system of any one of the previous aspects, wherein the testing apparatus further comprises a mixing module for combining produce milled into powder with a liquid to prepare a sample suspension therefrom.
22. The testing system of aspect 21, wherein the mixing module comprises a liquid source, at least one container, and a mixing motor;
   wherein the container receives the powder and the liquid, the container comprises at least one mixing implement, preferably wherein the implement is arranged to be driven by the mixing motor to mix the powder and liquid to create the sample suspension.
23. The testing system of aspect 22, wherein the mixing implement comprises a rotor with at least one blade, preferably with at least two blades.
24. The testing system of aspect 22 or 23, wherein the mixing implement is configured to move along a sidewall and/or a bottom wall of the container.
25. The testing system of any of aspects 21 to 24, wherein the mixing module further comprises one or more nozzles for injecting the liquid into the container, preferably wherein the one or more nozzles are arranged around a periphery of the container.
26. The testing system of aspect 25, wherein the liquid is delivered to the one or more nozzles at a pressure of at least 1 bar, preferably at least 2 bar, more preferably in the range of 3 to 4 bar.
27. The testing system according to any of aspects 21 to 26, further comprising a supernatant collector, preferably wherein the supernatant collector when actuated descends into the container to collect a supernatant of the suspension.
28. The testing system of aspect 27, wherein the supernatant collector comprises a mesh filter.
29. The testing system of aspect 28, wherein the supernatant collector is slidably affixed to the container and is in the form of a scoop comprising at least one wall formed forming the mesh filter.
30. The testing system of any of aspects 21 to 29, wherein the mixing module further comprises a turn table upon which the container is mounted, preferably wherein n containers, n being an integer of at least 2, 3 or 4, are mounted on the turn table.
31. The testing system of aspect 30, wherein the n containers are mounted on the turn table in a radially symmetric arrangement, preferably wherein the turn table is rotated by 360°/n degrees when actuated.
32. The grain testing system of aspect 31, wherein, n being an integer of at least 3, the containers rotate through at least three stations, including a mixing station, wherein the powder and liquid are received and the suspension is mixed, a testing station wherein the supernatant is collected and tested, and a cleaning station, wherein the container is rinsed.
33. The testing system of aspect 30, 31 or 32, wherein the turn table is rotated by a motor which is in electrical communication with and actuated by the control unit.
34. The testing system of any of aspects 21-33, wherein the mixing module further comprises an actuator configured to pivot the container such that the contents of the container are poured out due to gravity and to retract the container into the original position once empty.
35. The testing system of any one of aspects 21 to 34, wherein the testing apparatus further comprises a testing arrangement for performing a test on the sample suspension.
36. The testing system of aspect 35, wherein the testing arrangement further comprises an assay cartridge comprising a plurality of testing assays.
37. The testing system of aspect 36, wherein the testing arrangement further comprises a robotic arm for manipulating the assays, preferably wherein the robotic arm is configured to pick up an assay, introduce the assay into a supernatant of the suspension.
38. The testing system of aspect 37, wherein the robotic arm is configured to position the assay for read out, preferably outside the supernatant.
39. The testing system of any of aspects 36 to 38, wherein each assay comprises at least one lateral flow test, preferably a plurality of lateral flow tests, more preferably wherein the plurality of lateral flow tests are provided in a comb arrangement.
40. The testing system of any of aspects 36 to 39, wherein each assay comprises at least one nucleic acid amplification test, preferably a plurality of nucleic acid amplification tests, more preferably wherein a plurality of nucleic acid amplification tests are provided in a PCR strip arrangement.
41. The testing system of any of aspects 36 to 40, wherein the robotic arm further comprises a manipulator for handling the assays, preferably wherein the manipulator is a suction cup.
42. The testing system of any of aspects 36 to 41, wherein the testing arrangement further comprises a cartridge loader.
43. The testing system of aspect 42, wherein the cartridge loader further comprises a biasing mechanism such that when an assay is removed from the cartridge the next assay is moved into position for utilization, preferably upward.
44. The testing system of any of the previous aspects, wherein the testing arrangement further comprises a read-out arrangement for reading out the assay after use, preferably wherein the readout arrangement includes an imaging device for imaging the assay after testing.
45. The testing system of aspect 44, wherein the imaging device is actuated by the control unit and the test data comprises the imaging data.
46. The testing system of any one of the previous aspects, wherein the control unit further comprises a data processing, encryption and private server/ cloud connection unit for transmitting the test data and the test record.
47. The testing system of any one of the previous aspects, wherein the remote validation system further comprises one or more algorithms for evaluation of the test data and the test record which decides whether a validation certificate can be issued, preferably wherein one or more of these algorithms are image recognition and/or artificial intelligence algorithms.
48. The testing system of any one of the previous aspects, wherein the testing apparatus further comprises a user interface in connection with the control unit, the user interface being configured to enable a user to operate the testing system.
49. The grain testing system of aspect 48, wherein the user interface is further configured to display the validation.
50. The testing system of any one of the previous aspects, wherein the testing apparatus further comprises a cleaning module for cleaning and/or sanitizing the mixing module.
51. The testing system of aspect 50, wherein the cleaning module comprises a water source, a nozzle in communication with the water source for directing water into a container, and an outflow for funneling runoff from the container.
52. The testing system of aspect 51, wherein the cleaning module further comprises a decanter for collecting the runoff, preferably wherein the decanter is configured to separate solid materials from liquid.
53. A method for testing and validating an agricultural produce in the form of grain, oilseeds, legume pulses, beans and/or nuts at a user's testing site, the method comprising the steps of:
   i. receiving an amount of produce within a testing apparatus;
   ii. processing the produce with the testing apparatus;
   iii. testing the produce with the testing apparatus;
   iv. recording a testing record and a test data;
   v. transmitting the test data and the test record to a remote validation system;
   vi. validating the test data and the test record using the remote validation system; and
   vii. transmitting the validation to the user and/or a third party.
54. The method of aspect 53, wherein processing the grain includes measuring a predetermined amount of grain from the input amount; grinding the predetermined amount of grain into powder; mixing the powder with a liquid to create a suspension; and preferably sampling a supernatant of the suspension.
55. The method of aspect 54, wherein the predetermined amount is between 100 and 10000 individual grains, legume pulses or nuts, preferably between 500 and 2000, more preferably around 1000.
56. The method of aspect 54, wherein the predetermined amount is between 100 g and 1000 g, preferably between 200 g and 800 g, more preferably around 300 g.
57. The method of any of aspects 53 to 56, wherein testing the produce includes introducing an assay into a supernatant of a produce suspension.
58. The method of aspect 57, wherein recording the testing process and the test data further comprises recording a result of the assay with an imaging device and recording the operation of the testing apparatus with at least a second imaging device.
59. The method of any of aspects 53 to 58, wherein transmitting the test data further comprises encrypting and sending the test record and test data using a private cloud connection.
60. The method of any of aspects 53 to 59, wherein validating the results and test record includes receiving the test data and the test record, analyzing the test data, analyzing the test record, validating the test data and the test record on the basis of the analyses, and issuing and transmitting a validation certificate to the user or a third party.
61. The method of any of aspects 53 to 60, wherein an algorithm is used for evaluation of the test data and the test record which decides whether a validation certificate can be issued, preferably an image recognition and/or artificial intelligence algorithm.
62. The method of any of aspects 53 to 61, wherein the method is configured for testing produce for the presence of genetic modifications, preferably corn.
63. The method of any of aspects 53 to 62, wherein the method may be carried out for multiple different samples simultaneously.

## Claims

1. A testing system for an agricultural produce in the form of grain, oilseeds, legume pulses, beans and/or nuts, the test system comprising:
a testing apparatus for installation at a user's testing site and being configured to process and
test the produce, the testing apparatus comprising
one or more sensor devices configured to record the testing process as a test record, and
a control unit configured to operate the testing apparatus and transmit the test record and test data conveying a test result, and
a remote validation system comprising
a remote server configured to receive the test data and the test record from the testing apparatus;
wherein the remote validation system is configured to validate the test data and the test record and to transmit a validation to the user and/or a third party in the form of a certificate and/or test report.

2. The testing system of claim 1, wherein the testing system is configured for testing corn, and/or wherein the testing system is configured for detecting the presence of genetically modified produce.

3. The testing system of claim 1 or claim 2, wherein the testing apparatus is configured to grind the produce into powder, mix the powder with a liquid to form a mixture, test a sub-sample of the mixture, preferably a supernatant of the mixture, and transmit the test data and the test record to the remote validation system.

4. The testing system of any one of the previous claims, wherein the remote validation system is configured to:
receive the test data and the test record,
analyze the test data,
analyze the test record,
validate the test data and the test record on the basis of the analyses, and
issue and transmit a validation certificate to the user and/or third party.

5. The testing system of any one of the previous claims, wherein the testing apparatus is configured to receive an input of at least 100 g of produce, preferably at least 200 g of produce, more preferably at least 300 g of produce, and/or wherein the testing apparatus is configured to receive an input of at most 10 kg of produce , preferably at most 3 kg of produce , more preferably at most 2.5 kg of produce, and/or wherein the testing apparatus is configured to utilize at least 100 g, at least 200 g, or at least 300 g of corn for testing.

6. The testing system of any one of the previous claims, wherein the testing apparatus further comprises a feeder module for providing a randomized sample of the produce filled into the testing apparatus, the randomized sample having a predetermined weight and/or volume.

7. The testing system of any one of the previous claims, wherein the testing apparatus further comprises a grinding module for milling the produce, wherein the grinding module comprises a mill for grinding the produce into a powder, preferably wherein grinding module further comprises an accumulator arranged before the mill, more preferably above the mill, even more preferably wherein the produce moves from the accumulator into the mill by gravity.

8. The testing system of any one of the previous claims, wherein the testing apparatus further comprises a mixing module for combining produce milled into powder with a liquid to prepare a sample suspension therefrom.

9. The testing system of claim 8,
wherein the mixing module comprises a liquid source, at least one container, and a mixing motor;
wherein the container receives the powder and the liquid, the container comprises at least one mixing implement, preferably wherein the implement is arranged to be driven by the mixing motor to mix the powder and liquid to create the sample suspension.

10. The testing system of any of claim 8 or claim 9, wherein the mixing module further comprises a turn table upon which the container is mounted, preferably wherein *n* containers, *n* being an integer of at least 2, 3 or 4, are mounted on the turn table, wherein the n containers are mounted on the turn table in a radially symmetric arrangement, preferably wherein the turn table is rotated by 360°/n degrees when actuated and/or wherein, n being an integer of at least 3, the containers rotate through at least three stations, including a mixing station, wherein the powder and liquid are received and the suspension is mixed, a testing station wherein the supernatant is collected and tested, and a cleaning station, wherein the container is rinsed.

11. The testing system of any one of claims 8 to 10, wherein the testing apparatus further comprises a testing arrangement for performing a test on the sample suspension, preferably wherein the testing arrangement further comprises a robotic arm for manipulating the assays, more preferably wherein the robotic arm is configured to pick up an assay and introduce the assay into a supernatant of the suspension.

12. The testing system of any of claims 8 to 11, wherein each assay comprises at least one lateral flow test, preferably a plurality of lateral flow tests, more preferably wherein the plurality of lateral flow tests are provided in a comb arrangement.

13. The testing system of any one of the previous claims, wherein the control unit further comprises a data processing, encryption and private server/cloud connection unit for transmitting the test data and the test record.

14. A method for testing and validating an agricultural produce in the form of grain, oilseeds, legume pulses, beans and/or nuts at a user's testing site, the method comprising the steps of:
i. receiving an amount of produce within a testing apparatus;
ii. processing the produce with the testing apparatus;
iii. testing the produce with the testing apparatus;
iv. recording a testing record and a test data;
v. transmitting the test data and the test record to a remote validation system;
vi. validating the test data and the test record using the remote validation system; and
vii. transmitting the validation to the user and/or a third party.

15. The method of claim 14,
wherein processing the grain includes measuring a predetermined amount of grain from the input amount; grinding the predetermined amount of grain into powder; mixing the powder with a liquid to create a suspension; and preferably sampling a supernatant of the suspension, and/or
wherein testing the produce includes introducing an assay into a supernatant of a produce suspension, and/or
wherein transmitting the test data further comprises encrypting and sending the test record and test data using a private cloud connection.
